# EUROPEAN PATENT APPLICATION

(11) **EP 4 446 322 A1**
(43) Date of publication of application: **16.10.2024**
(21) Application number: 23742865.1
(22) Date of filing: 17.01.2023
(51) Int. Cl.: C07D 471/04, A61K 31/427, A61P 19/02

(54) **CYCLOPROPANAMIDE-CONTAINING COMPOUND AND USE THEREOF**

(30) Priority: 18.01.2022 CN 202210052435
(71) Applicant: CGeneTech (Suzhou, China) Co., Ltd., Suzhou, Jiangsu 215123 (CN)
(72) Inventor: WANG, Tong, Suzhou, Jiangsu 215123 (CN); HAO, Yan, Suzhou, Jiangsu 215123 (CN); XU, Yudong, Suzhou, Jiangsu 215123 (CN); YU, Qiang, Suzhou, Jiangsu 215123 (CN); DING, Juping, Suzhou, Jiangsu 215123 (CN); WANG, Yongsheng, Suzhou, Jiangsu 215123 (CN); CHEN, Bin, Suzhou, Jiangsu 215123 (CN); ZHAO, Jiahong, Suzhou, Jiangsu 215123 (CN)
(74) Representative: LLR
(86) International application number: PCT/CN2023/072506
(87) International publication number: WO 2023/138550

(57) **Abstract**

The present application relates to the field of pharmaceutical chemistry, and specifically to cyclopropanecarboxamide-contaning compounds of formula (I) and application thereof. The cyclopropanecarboxamide-containing compounds have great medical value and market potential for autoimmune diseases and myeloproliferative neoplasm diseases.

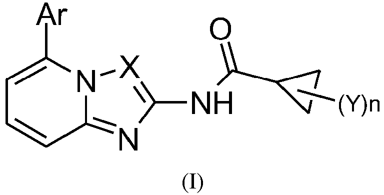

## Description

### Cross-Reference to Related Applications

The present application claims priority to Chinese patent application No. 202210052435.5 filed on January 18, 2022 and entitled "Cyclopropanecarboxamide-containing compounds and application thereof", the entire contents of which are incorporated herein by reference.

### Technical Field

The present application relates to the field of pharmaceutical chemistry, and specifically to cyclopropanecarboxamide-containing compounds and application thereof.

### Background Art

Protein kinases are a family of enzymes that catalyze the phosphorylation of specific residues in proteins and are broadly classified as tyrosine and serine/threonine kinases. Inappropriate kinase activities due to mutation, overexpression or inappropriate regulation, abnormal regulation or dysregulation, and overproduction or underproduction of growth factors or cytokines, are associated with a number of diseases including, but not limited to, cancer, cardiovascular diseases, anaphylaxis, asthma and other respiratory diseases, autoimmune diseases, inflammatory diseases, bone diseases, metabolic disorders, and neurological and neurodegenerative disorders (e.g., Alzheimer's disease). Therefore, protein kinases have emerged as an important class of enzymes as targets for therapeutic intervention.

Janus kinase (JAK) family is a class of non-receptor protein tyrosine kinases that play important roles in cytokine receptor signaling pathways through their interaction with signal transducer and activators of transcription (STAT). STAT is a group of cytoplasmic proteins that bind to DNA in the regulatory region of a target gene. As a downstream substrate of JAK, STAT can be activated by tyrosine phosphorylation under the stimulation of external signals, and then transferred into the nucleus to regulate gene transcription.

Regulations of many aberrant immune responses, such as allergies, asthma, allograft rejection, autoimmune diseases, including rheumatoid arthritis, amyotrophic lateral sclerosis and multiple sclerosis, as well as hematologic malignancies, including myeloproliferative disorders, leukemias, and lymphomas, are related to the JAK/STAT signaling pathway.

The Janus kinase (JAK) family includes four members, JAK1, JAK2, JAK3, and TYK2. JAK1, JAK2 and TYK2 are widely found in various tissues and cells, while JAK3 is mainly found in lymphocytes.

JAK1 can bind to IL-10, IL-19, IL-20, IL-22, IL-26, IL-28, IFN-a, IFN-γ, IL-6 of the gp130 family, and other yc-containing receptors, and the like (Rodig S. J., et al. Cell, 1998, 93:373-383). JAK1 is a novel target in the field of diseases, such as immune-related diseases, inflammations, and cancers. JAK1 inhibitors can be used to treat/prevent autoimmune diseases, inflammations (Homakova T., et al., Blood, 2010, 115:3287-3295), such as leukemia, lymphoma, melanoma, arthritis, psoriasis, lupus erythematosus, acquired immune deficiency syndrome (Hou S., et al., HµM. Genet., 2013,132:1049-1058), and the like.

JAK2 plays an important role in the regulation of multiple receptor signals including IL-3, IFN-γ, EPO, GH, and the like (Levy D. E., et al., Nat. Rev. Mol. Cell Biol., 2002, 3:651-662). JAK2 inhibitors have great medical value and market potential for the treatment of diseases such as myelofibrosis, solid tumors, and hematological tumors.

JAK3 regulates cell signaling by binding to the common γ-chain (γc) in the cytokine receptor complexes such as IL-2, IL-4, IL-7, IL-9, IL-15, and IL-21. JAK3 inhibitors can block T-cell activation and prevent graft rejection after transplantation. In addition, JAK3 inhibitors also can regulate the functions of lymphocytes, macrophages, and mast cells. JAK3 inhibitors are expected for the treatment or prevention of a variety of diseases involving the functions of lymphocytes, macrophages, or mast cells.

Tofacitinib is the first new orally available JAK inhibitor approved by the FDA that acts on JAK1 and JAK3 for the treatment of rheumatoid arthritis (RA). However, tofacitinib may bring some side effects along with the relief of RA symptoms, triggering the developments of certain infections, malignancies, and lymphomas. Studies have shown that the overall risk of infection and mortality with tofacitinib is similar to that of biologics for treating RA. Considering the pleiotropy of JAK in many regulatory pathways and immune processes, non-selective JAK inhibitors will bring the risk of adverse effects, and selective JAK inhibitors have become an important direction in the current research.

Filgotinib from Galapagos, Belgium, is a new generation of JAK1-selective inhibitors with the potential to reduce the risk of anemia or infection caused by tofacitinib. However, Filgotinib has relatively weak activity, IC₅₀ against JAK1 thereof is greater than 10 nM, and the clinical dosage is relatively high (Expert Opin. Investig. Drugs. 2016, 25, 1355).

Although a series of JAK inhibitors have been disclosed in the market, there remains a need to develop new compounds with better efficacy and JAK selectivity, and through continuous efforts, the present application develops a compound having the structure of the formula (I), and compounds having such structures are found to exhibit excellent effects.

### Summary of the Invention

The present application is aimed to provide cyclopropanecarboxamide-containing compounds. The cyclopropanecarboxamide-containing compounds are selected from: a compound of formula (I), a stereoisomer, or a pharmaceutically acceptable salt thereof. The stereoisomer includes an enantiomer, a diastereomer, a mesomer or a racemate, etc.

In order to achieve the object of the present application, the following technical solutions are employed in the present application.

The present application provides a compound of formula (I), a stereoisomer, or a pharmaceutically acceptable salt thereof: 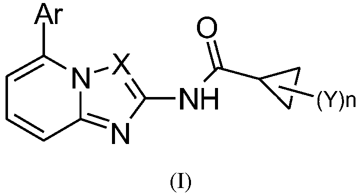 wherein,
X is selected from -CH or N;
Y is selected from hydrogen or halogen, and n is 1 or 2;
Ar is selected from 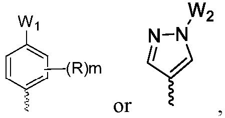 wherein:
m is an integer from 1-4;
Wi is selected from 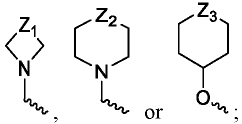
Wz is selected from 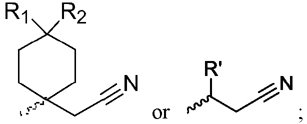
R is selected from hydrogen, C₁-C₆ alkyl, C₁-C₆ alkoxy, or halogen;
Z₁, Z₂ and Z₃ are each independently selected from -C(R₃R₄)-, -S(O)₂-, -S(O)-, -CH(S(O)₂R')-, or - CH(S(O)₂NR'R")-;
R₁ and R₂ are each independently selected from hydrogen, halogen, C₁-C₆ alkyl, C₁-C₆ haloalkyl, or C₃-C₇ cycloalkyl;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(Ci-Ce alkyl)₂;
R₃ and R₄ are each independently selected from hydrogen, halogen, C₁-C₆ alkyl, C₁-C₆ haloalkyl, or C₃-C₇ cycloalkyl.

Preferably, the compound, the stereoisomer, or the pharmaceutically acceptable salt thereof, has a structure of formula (II): 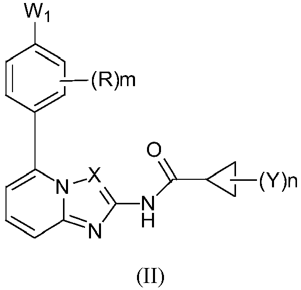 wherein,
X is selected from -CH or N;
Y is selected from hydrogen or halogen, and n is 1 or 2;
m is an integer from 1-4;
Wi is selected from 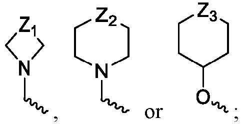
R is selected from hydrogen, C₁-C₆ alkyl, C₁-C₆ alkoxy, or halogen;
Z₁, Z₂ and Z₃ are each independently selected from -C(R₃R₄)-, -S(O)₂-, -S(O)-, -CH(S(O)₂R')-, or - CH(S(O)₂NR'R")-, and when Y is hydrogen, Z₂ is not -S(O)₂-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(Ci-Ce alkyl)₂;
R₃ and R₄ are each independently selected from hydrogen, halogen, C₁-C₆ alkyl, C₁-C₆ haloalkyl, or C₃-C₇ cycloalkyl.

Preferably, the compound, the stereoisomer, or the pharmaceutically acceptable salt thereof, has a structure of formula (III): 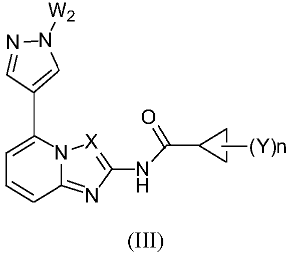 wherein,
X is selected from- CH or N;
Y is selected from hydrogen or halogen, and n is 1 or 2;
Wz is selected from 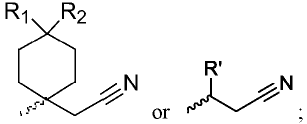
R₁ and R₂ are each independently selected from hydrogen, halogen, C₁-C₆ alkyl, C₁-C₆ haloalkyl, or C₃-C₇ cycloalkyl;
R' is selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂.

Preferably, the compound, the stereoisomer, or the pharmaceutically acceptable salt thereof, has a structure of formula (IV): 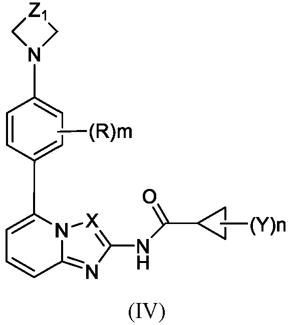 wherein,
X is selected from -CH or N;
Y is selected from hydrogen or halogen, and n is 1 or 2;
m is an integer from 1-4;
R is selected from hydrogen, C₁-C₆ alkyl, C₁-C₆ alkoxy, or halogen;
Z₁ is selected from -C(R₃R₄)-, -S(O)₂-, -S(O)-, -CH(S(O)₂R')-, or -CH(S(O)₂NR'R")-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(Ci-Ce alkyl)₂;
R₃ and R₄ are each independently selected from hydrogen, halogen, C₁-C₆ alkyl, C₁-C₆ haloalkyl, or C₃-C₇ cycloalkyl.

Preferably, the compound, the stereoisomer, or the pharmaceutically acceptable salt thereof, has a structure of formula (V): 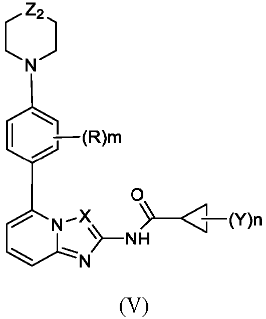 wherein,
X is selected from -CH or N;
Y is selected from hydrogen or halogen, and n is 1 or 2;
m is an integer from 1-4;
R is selected from hydrogen, C₁-C₆ alkyl, C₁-C₆ alkoxy, or halogen;
Z₂ is selected from -C(R₃R₄)-, -S(O)₂-, -S(O)-, -CH(S(O)₂R')-, or -CH(S(O)₂NR'R")-; and when Y is hydrogen, Z₂ is not -S(O)₂-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

Preferably, the compound, the stereoisomer, or the pharmaceutically acceptable salt thereof, has a structure of formula (VI): 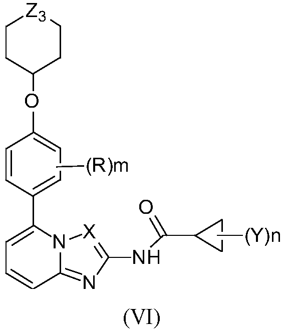 wherein,
X is selected from -CH or N;
Y is selected from hydrogen or halogen, and n is 1 or 2;
m is an integer from 1-4;
R is selected from hydrogen, C₁-C₆ alkyl, C₁-C₆ alkoxy, or halogen;
Z₃ is selected from -C(R₃R₄)-, -S(O)₂-, -S(O)-, -CH(S(O)₂R')-, or -CH(S(O)₂NR'R")-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

Preferably, the compound, the stereoisomer, or the pharmaceutically acceptable salt thereof, has a structure of formula (VII): 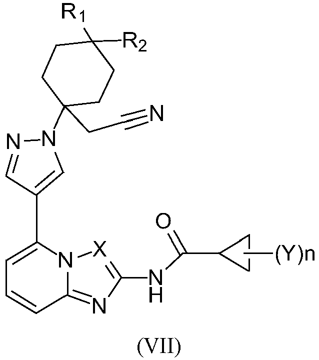 wherein,
X is selected from -CH or N;
Y is selected from hydrogen or halogen, and n is 1 or 2;
R₁ and R₂ are each independently selected from hydrogen, halogen, C₁-C₆ alkyl, C₁-C₆ haloalkyl, or C₃-C₇ cycloalkyl.

Preferably, the compound, the stereoisomer, or the pharmaceutically acceptable salt thereof, has a structure of formula (VIII): 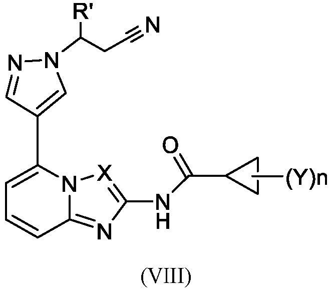 wherein,
X is selected from -CH or N;
Y is selected from hydrogen or halogen, and n is 1 or 2;
R' is selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂.

Preferably, Y in the above formulas is selected from H or F.

Preferably, 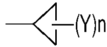 in the above formulas is selected from

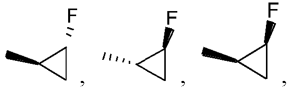

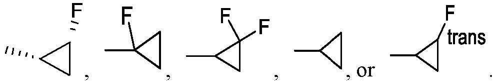

Preferably, in formula (IV), Z₁ is selected from -CH(SO₂R')-, wherein R' is selected from unsubstituted C₁-C₆ alkyl or C₃-C₇ cycloalkyl; R is selected from hydrogen, methyl, methoxy, or halogen.

Preferably, in formula (V), when Y is hydrogen, Z₂ is selected from -CR₃R₄- or -CH(S(O)₂R'), and further, wherein R₃ and R₄ are each independently selected from hydrogen, halogen, C₁-C₆ alkyl, C₁-C₆ haloalkyl, or C₃-C₇ cycloalkyl, R' is selected from C₁-C₆ alkyl or C₃-C₇ cycloalkyl, R is selected from hydrogen, methyl, methoxy or halogen; when Y is halogen, Z₂ is selected from -SO₂-, and R is selected from hydrogen, methyl, methoxy, or halogen.

Preferably, in formula (VI), Z₃ is selected from -SO₂- or -S(O)-; R is selected from hydrogen, methyl, methoxy or halogen.

Preferably, in formula (VII), R₁ and R₂ are each independently selected from hydrogen, fluorine, methyl, ethyl, n-propyl, isopropyl, n-butyl, isobutyl, tert-butyl, sec-butyl, n-pentyl, trifluoromethyl, cyclopropyl, or cyclopentyl.

Preferably, in formula (VIII), R' is selected from substituted or unsubstituted methyl, ethyl, n-propyl, isopropyl, n-butyl, isobutyl, tert-butyl, sec-butyl, n-pentyl, isopentyl, cyclopropyl, or cyclopentyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl and N(C₁-C₆ alkyl)₂.

Further, the present application also provides compounds having the following formulas, stereoisomers or pharmaceutically acceptable salts thereof:

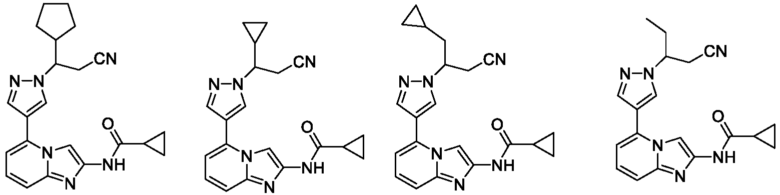

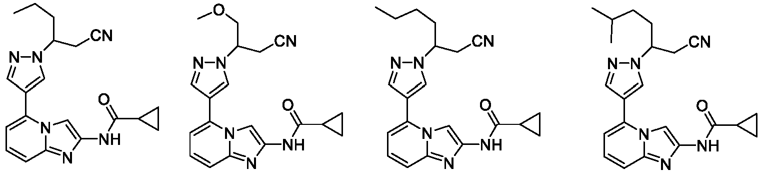

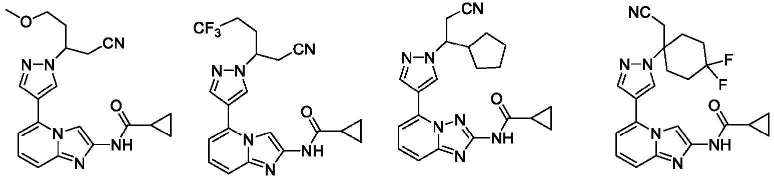

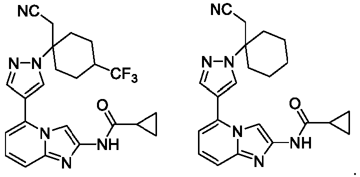

Further, the present application also provides compounds having the following formulas, stereoisomers or pharmaceutically acceptable salts thereof:

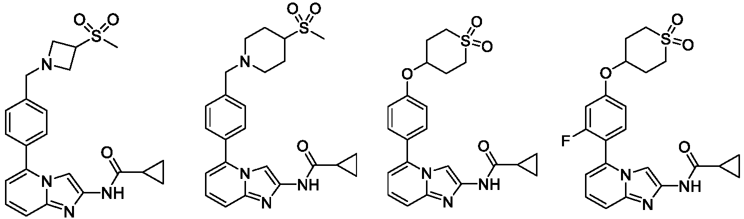

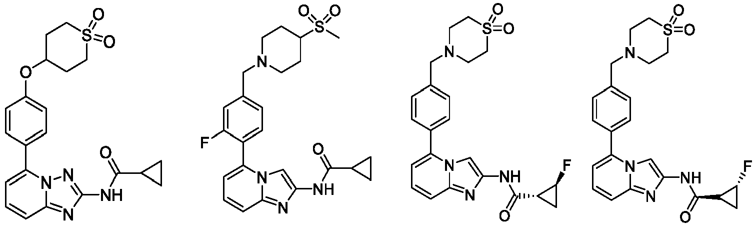

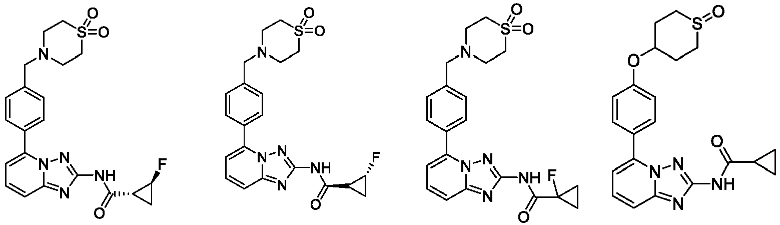

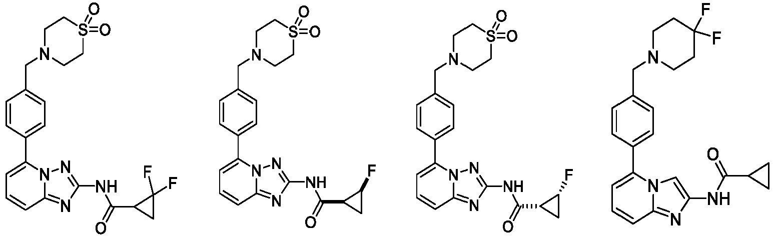

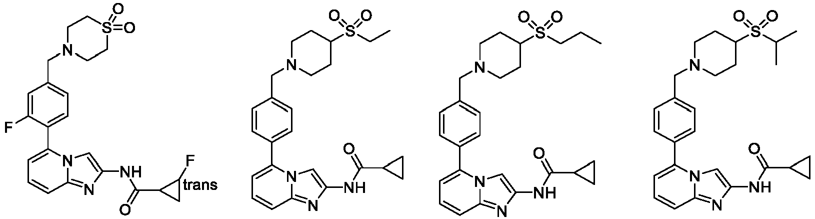

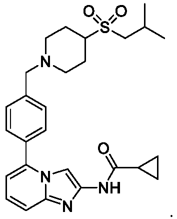

Further, the present application also provides a pharmaceutical composition, comprising a therapeutically effective amount of the compound, the stereoisomer, or the pharmaceutically acceptable salt thereof, and a pharmaceutically acceptable carrier.

Further, the present application also provides the use of the compound, the stereoisomer, or the pharmaceutically acceptable salt thereof, or the pharmaceutical composition in the preparation of a medicament for treating a disease associated with abnormal JAK signaling pathway.

Preferably, the disease is an autoimmune disease.

Further preferably, the autoimmune disease is selected from rheumatoid arthritis, ulcerative colitis, atopic dermatitis, or systemic lupus erythematosus.

Preferably, the disease is a myeloproliferative neoplasm disease.

Further preferably, the myeloproliferative neoplasm disease is selected from essential thrombocytosis, polycythemia vera, or primary myelofibrosis diseases.

Further, the present application also provides a method for treating a disease associated with abnormal JAK signaling pathway, the method comprising: administering a therapeutically effective amount of the compound, the stereoisomer, or the pharmaceutically acceptable salt thereof, or the above pharmaceutical composition to a subject in need thereof.

Preferably, the disease is an autoimmune disease.

Further preferably, the autoimmune disease is selected from rheumatoid arthritis, ulcerative colitis, atopic dermatitis, or systemic lupus erythematosus.

Preferably, the disease is a myeloproliferative neoplasm disease.

Further preferably, the myeloproliferative neoplasm disease is selected from essential thrombocytosis, polycythemia vera, or primary myelofibrosis diseases.

The "therapeutically effective amount" may vary depending on the subject administered, the subject's organs, symptoms, methods of administration, etc., and may be determined by the physician's judgment, considering the type of dosage form, method of administration, age and weight and symptoms of the patient, etc.

### Beneficial effects

1) The compounds of the present application have good JAK1, JAK2 or JAK3 kinase inhibitory activity, and the compounds of the present application have significant selectivity for JAK1, JAK2 or JAK3 kinase.
2) The compounds of the present application have obvious pharmacokinetic advantages, which provide more options for the prevention and/or treatment of diseases associated with abnormal JAK signaling pathway, and have good prospects for clinical application.

### Detailed description of the invention

In order to make the purposes, technical solutions, and advantages of the examples of the present application clearer, the technical solutions in the examples of the present application are clearly and completely described below. Apparently, the examples described are a part of the examples of the present application, not all of the examples. Based on the examples in the present application, all other examples obtained by those of ordinary skill in the art without creative labor shall fall within the scope of protection of the present application.

It should be noted that the terms used in the description and claims have the following meanings, unless indicated to the contrary.

The term "alkyl" refers to a saturated aliphatic hydrocarbon group comprising straight and branched groups containing 1 to 20 carbon atoms, which is preferably alkyl containing 1 to 10 carbon atoms, more preferably alkyl containing 1 to 6 carbon atoms, most preferably alkyl containing 1 to 4 carbon atoms, and most preferably methyl.

Non-limiting examples include methyl, ethyl, n-propyl, isopropyl, n-butyl, isobutyl, *tert*-butyl, *sec-*butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferred is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted, and when substituted, a substituent may be at any available linkage point, and preferably the substituent is one or more independently selected from the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group.

The term "cycloalkyl" refers to a saturated or partially unsaturated monocyclic or polycyclic hydrocarbon substituent comprising 3 to 20 carbon atoms, preferably 3 to 12 carbon atoms, more preferably 3 to 10 carbon atoms, most preferably 3 to 7 carbon atoms, and most preferably cyclopropyl or cyclopentyl. Non-limiting examples of a monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, and preferably cyclopropyl and cyclopentyl. Polycyclic cycloalkyl includes cycloalkyl of a spiro ring, a fused ring, and a bridged ring. The cycloalkyl may be optionally substituted or unsubstituted, and when substituted, the substituent is preferably one or more independently selected from the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group.

The term "alkoxy" refers to -O-(alkyl) and -O-(unsubstituted cycloalkyl), wherein alkyl and cycloalkyl are as defined above. Non-limiting examples include methoxy, ethoxy, propoxy, butoxy, cyclopropoxy, cyclobutoxy, cyclopentyloxy, cyclohexyloxy, and the like. The alkoxy group may be substituted or unsubstituted, and when substituted, the substituent may be preferably one or more independently selected from the following group: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxyl, heterocycloalkoxyl, cycloalkylthio, heterocycloalkylthio, amino, haloalkyl, hydroxyalkyl, carboxyl, or a carboxylate group.

"Haloalkyl" refers to alkyl substituted by one or more halogens, wherein alkyl is as defined above. "Hydroxyl" refers to the -OH group.

"hydroxyalkyl" refers to alkyl substituted by hydroxyl, wherein alkyl is as defined above. "Halogen" refers to fluorine, chlorine, bromine or iodine, preferably fluorine or iodine.

"Amino" refers to -NH₂.

"Cyano" refers to -CN.

"Nitro" refers to -NO₂.

"Oxo" refers to =O.

"Carboxyl" refers to -C(O)OH.

The "carboxylate group" refers to -C(O)O (alkyl) or (cycloalkyl), wherein alkyl and cycloalkyl are as defined above.

"Optional" or "optionally" means that an event or environment described subsequently can but does not necessarily occur, and this description includes cases in which the event or environment occurs or does not occur. For example, a "heterocyclic alkyl group optionally substituted by an alkyl" means that the alkyl may but does not necessarily exist, and this description includes a situation that the heterocyclic alkyl group is substituted by the alkyl and a situation that the heterocyclic alkyl group is not substituted by the alkyl.

"Substituted" means that one or more hydrogen atoms in a group, preferably at most 5, and more preferably 1-3 hydrogen atoms, are independently substituted by a corresponding number of substituents. It is apparent that the substituents are only in their possible chemical positions, and those skilled in the art may determine (by experiments or theories) possible or impossible substitutions without excessive effort. For example, an amino or hydroxyl with a free hydrogen may be unstable when linked with carbon atoms with an unsaturated (such as ene) bond.

The technical solutions of the present application are further described in combination with specific examples as follows. Unless otherwise indicated, the instruments, consumables, reagents, etc., used in the following examples are available by conventional commercial means, and the experimental methods for which specific conditions are not indicated in the examples are selected in accordance with conventional methods and conditions or in accordance with instruction manuals.

### Example 1: Synthesis of Compounds 1-11

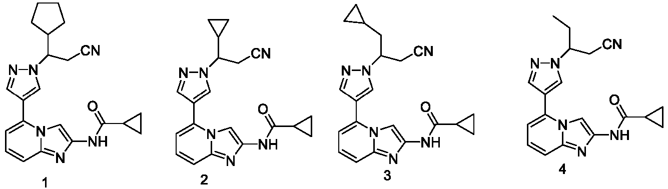

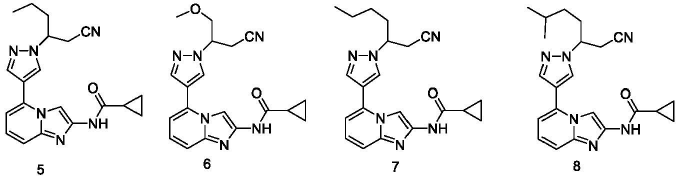

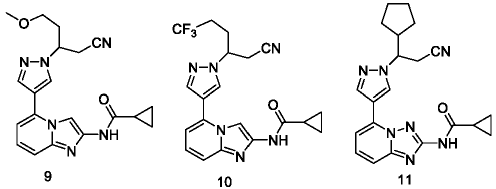

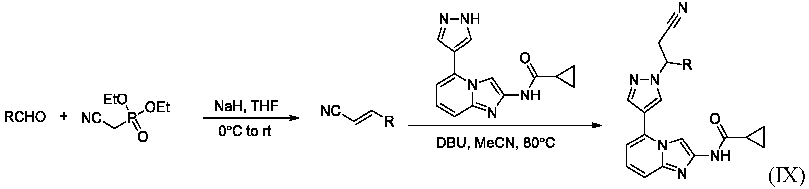

### Synthetic route for the compound of formula (IX) to which Compounds 1-11 belong

### Step 1:

Diethyl cyanomethylphosphonate (177 mg, 1 mmol) was dissolved in dry tetrahydrofuran (10 mL), in an ice bath, sodium hydride (60 mg, 1.5 mmol) was added and stirred for one hour before the corresponding aldehyde (1 mmol) was added. The reaction material was then stirred at room temperature overnight. After the reaction was completed, the reaction was quenched with a saturated ammonium chloride solution (20 mL), extracted with ethyl acetate (10 mL × 3), then the organic phases were combined and washed with saturated salt water (20 mL), dried with anhydrous sodium sulfate, filtered and concentrated under reduced pressure. The residue was used directly in the next step.

### Step 2:

N-(5-(1H-pyrazol-4-yl)imidazo[1,2-a]pyridin-2-yl)cyclopropanecarboxatnide (134 mg, 0.5 mmol), the residue from the previous step (0.6 mmol), and 1,8-diazabicyclo[5,4,0]undecene-7 (two drops) were dissolved in acetonitrile and heated to 80 °C for reacting for 8 hours. After restored to room temperature, the reactants were added with water (20 mL), concentrated to remove most acetonitrile, and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to give the compound.

### Example 2: Synthesis of Compound 1

In accordance with the synthetic route of Example 1, cyclopentyl formaldehyde was used in Step 1 to obtain Compound 1 (70 mg) in 36% yield. LCMS (ESI +):389.42 (M + H)⁺.

### Example 3: Synthesis of Compound 2

In accordance with the synthetic route of Example 1, cyclopropyl formaldehyde was used in Step 1 to obtain Compound 2 (100 mg) in 56% yield. LCMS (ESI +):361.42 (M + H)⁺.

### Example 4: Synthesis of Compound 3

In accordance with the synthetic route of Example 1, 2-cyclopropyl acetaldehyde was used in Step 1 to obtain Compound 3 (73 mg) in 39% yield. LCMS (ESI +):375.38 (M + H)⁺.

### Example 5: Synthesis of Compound 4

In accordance with the synthetic route of Example 1, propionaldehyde was used in Step 1 to obtain Compound 4 (80 mg) in 41% yield. LCMS (ESI +):349.16 (M + H)⁺.

### Example 6: Synthesis of Compound 5

In accordance with the synthetic route of Example 1, butyraldehyde was used in Step 1 to obtain Compound 5 (79 mg) in 43% yield. LCMS (ESI +):363.20 (M + H)⁺.

### Example 7: Synthesis of Compound 6

In accordance with the synthetic route of Example 1, 2-methoxy acetaldehyde was used in Step 1 to obtain Compound 6 (55 mg) in 30% yield. LCMS (ESI +):365.06 (M + H)⁺.

### Example 8: Synthesis of Compound 7

In accordance with the synthetic route of Example 1, valeraldehyde was used in Step 1 to obtain Compound 7 (72 mg) in 38% yield. LCMS (ESI +): 377.08 (M + H)⁺.

### Example 9: Synthesis of Compound 8

In accordance with the synthetic route of Example 1, 4-methyl valeraldehyde was used in Step 1 to obtain Compound 8 (87 mg) in 46% yield. LCMS (ESI +):377.12 (M + H)⁺.

### Example 10: Synthesis of Compound 9

In accordance with the synthetic route of Example 1, 3-methoxy propionaldehyde was used in Step 1 to obtain Compound 9 (80 mg) in 42% yield. LCMS (ESI +):379.09 (M + H)⁺.

### Example 11: Synthesis of Compound 10

In accordance with the synthetic route of Example 1, 4,4,4-trifluorobutyraldehyde was used in Step 1 to obtain Compound 10 (105 mg) in 50% yield. LCMS (ESI +):417.16 (M + H)⁺.

### Example 12: Synthesis of Compound 11

In accordance with the synthetic route of Example 1 except that N-(5-(1H-pyrazol-4-yl)-[1,2,4]triazolyl[1,5-a]pyridin-2-yl)cyclopropanecarboxamide was used instead of N-(5-(1H-pyrazol-4-yl)imidazo[1,2-a]pyridin-2-yl)cyclopropanecarboxamide in Step 2, and cyclopentyl formaldehyde was used in Step 1 to obtain Compound 11 (79 mg) in 41% yield. LCMS (ESI +):390.25 (M + H)⁺.

### Example 13: Synthesis of Compounds 12,13, 14

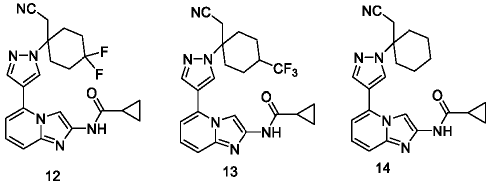

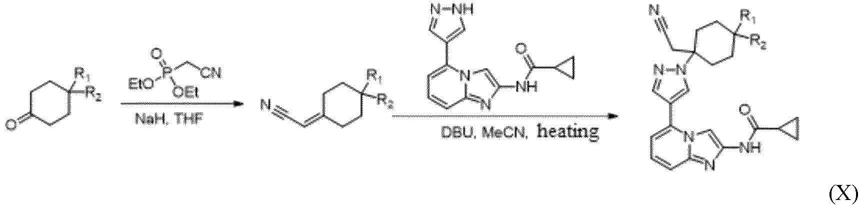

### Synthetic route for the compound of formula (X) to which Compounds 12, 13, 14 belong

### Step 1:

Diethyl cyanomethylphosphonate (177 mg, 1 mmol) was dissolved in dry tetrahydrofuran (10 mL), in an ice bath, sodium hydride (60 mg, 1.5 mmol) was added and stirred for one hour before a cyclohexanone derivative (1 mmol) was added. The reaction material was then stirred at room temperature overnight. The reaction was quenched with a saturated ammonium chloride solution (20 mL), extracted with ethyl acetate (20 mL × 3), then the organic phases were combined and washed with saturated salt water (20 mL), dried with anhydrous sodium sulfate, filtered and concentrated under reduced pressure. The resulting residue was used directly in the next step.

### Step 2:

N-(5-(1H-pyrazol-4-yl)imidazo[1,2-a]pyridin-2-yl)cyclopropanecarboxamide (135 mg, 0.5 mmol), the residue from Step 1 (0.6 mmol), and 1,8-diazabicyclo[5,4,0]undecene-7 (two drops) were dissolved in acetonitrile and heated to 80 °C for reacting for 8 hours. After restored to room temperature, the reactants were added with water (20 mL), concentrated to remove most acetonitrile, and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to give the compound.

### Example 14: Synthesis of Compound 12

In accordance with the synthetic route of Example 13, 4,4-difluorocyclohexanone was used in Step 1 to obtain Compound 12 (74 mg) in 35% yield. LCMS (ESI +):425.02 (M + H)⁺.

### Example 15: Synthesis of Compound 13

In accordance with the synthetic route of Example 13, 4-trifluoromethylcyclohexanone was used in Step 1 to obtain Compound 13 (113 mg) in 49% yield. LCMS (ESI +): 457.01 (M + H)⁺.

### Example 16: Synthesis of Compound 14

In accordance with the synthetic route of Example 13, cyclohexanone was used in Step 1 to obtain Compound 14 (99 mg) in 50% yield. LCMS (ESI +): 389.08 (M + H)⁺.

### Example 17: Synthesis of Compound 15

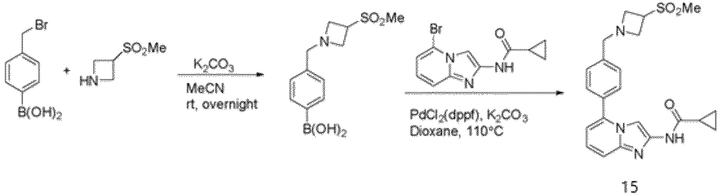

### Step 1:

(4-(bromomethyl)phenyl)-boronic acid (215 mg, 1 mmol) and 3-(methylsulfonyl)azetidine (135 mg, 1 mmol) were dissolved in acetonitrile, followed by potassium carbonate (207 mg, 1 mmol) in batches. The reaction was carried out at room temperature overnight. The reactants were filtered and the filtrate was concentrated. The resulting residue was used directly in the next step.

### Step 2:

The residue obtained in the previous step (135 mg, 0.5 mmol), N-(5-bromoimidazo[1,2-a]pyridin-2-yl)cyclopropanecarboxamide (140 mg, 1 mmol), [1,1'-bis(diphenylphosphino)ferrocene]palladium dichloride (36 mg, 0.1 mmol) and potassium carbonate (138 mg, 2 mmol) were dissolved in dioxane (10 mL) and water (10 mL). The reaction material was subject to 3 times of nitrogen displacement and then stirred at 110 °C for 8 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain Compound 15 (119 mg) in 56% yield. LCMS (ESI +): 212.99 (M/2 + H)⁺.

### Example 18: Synthesis of Compound 16

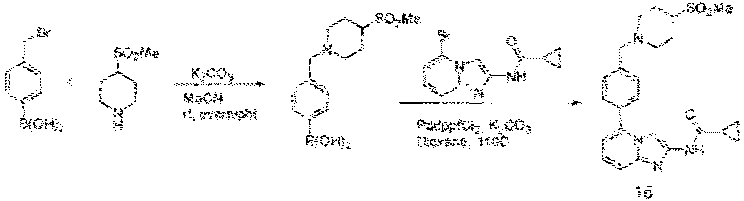

### Step 1:

(4-(bromomethyl)phenyl)-boronic acid (108 mg, 0.5 mmol) and 4-(methylsulfonyl)piperidine (84 mg, 0.5 mmol) were dissolved in acetonitrile (5 mL), followed by potassium carbonate (138 mg, 1 mmol) in batches. The reaction was carried out at room temperature overnight. The reactants were filtered and the filtrate was concentrated. The resulting residue was used directly in the next step.

### Step 2:

The residue obtained in Step 1 was dissolved in dioxane (10 mL) and water (10 mL), followed by adding N-(5-bromoimidazo[1,2-a]pyridin-2-yl)cyclopropanecarboxatnide (140 mg, 0.5 mmol), [1,1'-bis(diphenylphosphino)ferrocene]palladium dichloride (36 mg, 0.1 mmol) and potassium carbonate (138 mg, 2 mmol). The reaction material was subject to 3 times of nitrogen displacement and then stirred at 110 °C for 8 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain Compound 16 (108 mg) in 48% yield. LCMS (ESI +): 227.13 (M/2 + H)+.

### Example 19: Synthesis of Compound 17

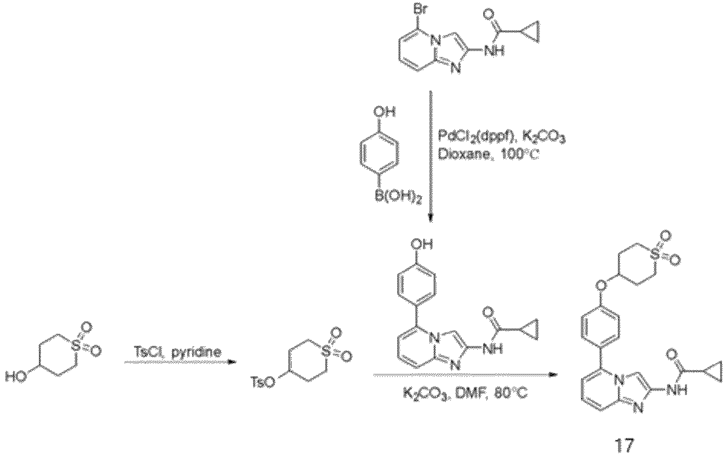

### Step 1:

Tetrahydro-2H-thiopyran-4-ol-1,1-dioxide (150 mg, 1 mmol) andp-toluenesulfonyl chloride (215 mg, 1 mmol) were dissolved in pyridine (5 mL) and reacted overnight at room temperature. After concentration, the residue was purified by column chromatography (petroleum ether:ethyl acetate = 100:0-50:50) to obtain a compound 1,1-dioxidotetrahydro-2H-thiopyran-4-yl-4-methylbenzenesulfonate.

### Step 2:

*P*-hydroxyphenylboronic acid (138 mg, 1 mmol), N-(5-bromoimidazo[1,2-a]pyridin-2-yl)cyclopropanecarboxamide (279 mg, 1 mmol), [1,1'-bis(diphenylphosphino)ferrocene]palladium dichloride (73 mg, 0.1 mmol) and potassium carbonate (276 mg, 2 mmol) were dissolved in dioxane (10 mL) and water (10 mL). The reaction material was subject to 3 times of nitrogen displacement and then stirred at 100 °C for 8 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain a compound N-(5-(4-hydroxyphenyl)imidazo[1,2-a]pyridin-2-yl)cyclopropanecarboxamide.

### Step 3:

To N,N-dimethylformamide (5 mL), was added 1,1-dioxidotetrahydro-2H-thiopyran-4-yl-4-methylbenzenesulfonate (152 mg, 0.5 mmol) obtained in Step 1, N-(5-(4-hydroxyphenyl)imidazo[1,2-a]pyridin-2-yl)cyclopropanecarboxatnide (146 mg, 0.5 mmol) obtained in Step 2 and potassium carbonate (138 mg, 1 mmol). The reaction material was heated to 80 °C for 5 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain Compound 17 (137 mg) in 62% yield. LCMS (ESI +): 425.98 (M + H)⁺.

### Example 20: Synthesis of Compound 18

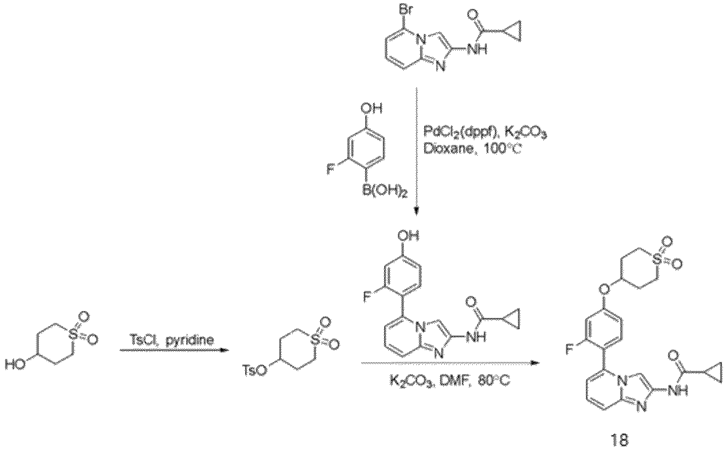

In accordance with the synthetic route of Example 19, 2-fluoro-4-hydroxyphenylboronic acid was used instead of *p*-hydroxyphenylboronic acid in Step 2 to obtain Compound 18 (104 mg) in 50% yield. LCMS (ESI +): 443.98 (M + H)⁺.

### Example 21: Synthesis of Compound 19

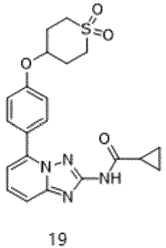

In accordance with the synthetic route of Example 19, N-(5-bromo-[1,2,4]triazolo[1,5-a]pyridin-2-yl)cyclopropanecarboxamide was used instead of N-(5-bromoimidazo[1,2-a]pyridin-2-yl)cyclopropanecarboxamide in Step 2 to obtain Compound 19 (58 mg) in 43% yield. LCMS (ESI +): 427.15 (M + H)⁺

### Example 22: Synthesis of Compound 20

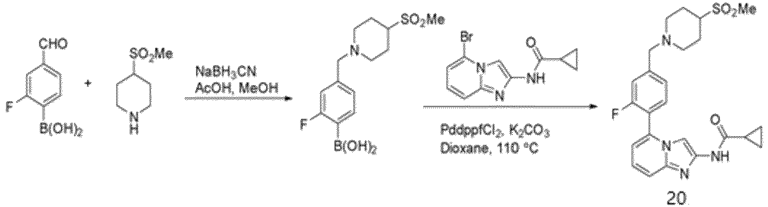

### Step 1:

To methanol, was added (2-fluoro-4-formylphenyl)boronic acid (168 mg, 1 mmol), 4-(methylsulfonyl)piperidine (135 mg, 1 mmol), and a drop of acetic acid. After stirring at room temperature for two hours, sodium cyanoborohydride (126 mg, 2 mmol) was added followed by stirring overnight. The reactants were concentrated and added with water (20 mL) and then extracted with ethyl acetate (10 mL × 3). The organic phases were then combined and washed with saturated salt water (20 mL), dried with anhydrous sodium sulfate, filtered and concentrated under reduced pressure, and the resulting residue was used directly in the next step.

### Step 2:

The residue obtained in Step 1 was directly dissolved in dioxane (10 mL) and water (10 mL), followed by adding N-(5-bromoimidazo[1,2-a]pyridin-2-yl)cyclopropanecarboxamide (140 mg, 0.5 mmol), [1,1'-bis(diphenylphosphino)ferrocene]palladium dichloride (36 mg, 0.05 mmol) and potassium carbonate (138 mg, 1 mmol). The reaction material was subjected to 3 times of nitrogen displacement and then stirred at 100 °C for 8 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain Compound 20 (120 mg) in 51% yield. LCMS (ESI +): 236.13 (M/2 + H)⁺.

### Example 23: Synthesis of Compound 21

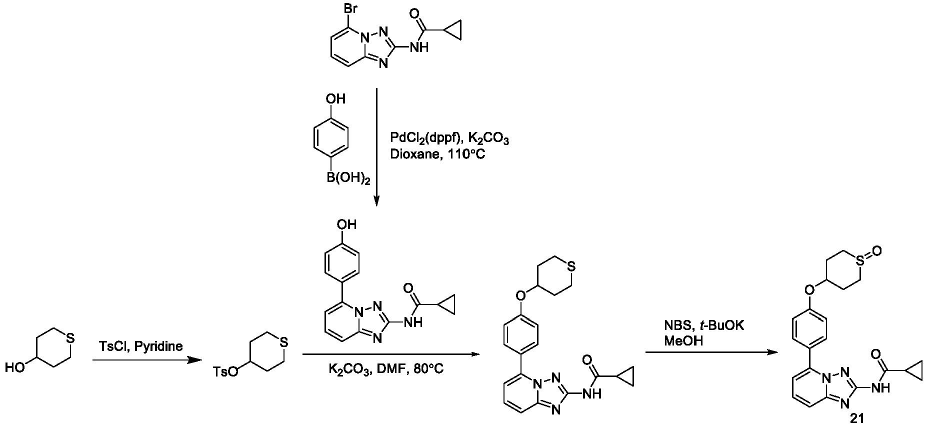

### Step 1:

Tetrahydrothiopyran-4-ol (590 mg, 5 mmol) and p-toluenesulfonyl chloride (1.1 g, 6 mmol) were dissolved in pyridine (10 mL) and reacted at room temperature overnight. After concentration, the residue was purified by column chromatography (petroleum ether:ethyl acetate = 100:0-50:50) to give a compound tetrahydro-2H-thiopyran-4-yl-4-methylbenzenesulfonate.

### Step 2:

P-hydroxyphenylboronic acid (1.38 g, 10 mmol), N-(5-bromo-[1,2,4]triazolo[1,5-a]pyridin-2-yl)cyclopropanecarboxamide (2.81 g, 10 mmol), [1,1'-bis(diphenylphosphino)ferrocene]palladium dichloride (730 mg, 1 mmol) and potassium carbonate (2.76 g, 20 mmol) were dissolved in dioxane (20 mL) and water (20 mL). The reaction material was subject to 3 times of nitrogen displacement and then stirred at 110 °C for 8 hours. After restored to room temperature, the reactants were added with water (20 mL) and then extracted with ethyl acetate (20 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain a compound N-(5-(4-hydroxyphenyl)-[1,2,4]triazolo[1,5-a]pyridin-2-yl)cyclopropanecarboxamide.

### Step 3:

To N,N-dimethylformamide (5 mL), was added tetrahydro-2H-thiopyran-4-yl-4-methylbenzenesulfonate (272 mg, 1 mmol) obtained in Step 1, N-(5-(4-hydroxyphenyl)-[1,2,4]triazolo[1,5-a]pyridin-2-yl)cyclopropanecarboxatnide (294 mg, 1 mmol) obtained in Step 2 and potassium carbonate (276 mg, 2 mmol). The reaction material was heated to 80 °C and reacted for 5 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to give a compound N-(5-(4-((1-tetrahydrothiopyran-4-yl)oxy)phenyl)-[1,2,4]triazolo[1,5-a]pyridin-2-yl)cyclopropanecarboxamide. N-(5-(4-((1-tetrahydrothiopyran-4-yl)oxy)phenyl)-[1,2,4]triazolo[1,5-a]pyridin-2-yl)cyclopropanecarboxamide (197 mg, 1 mmol) was dissolved in methanol (5 mL), and then N-bromosuccinimide (178 mg, 2 mmol) and potassium *tert*-butoxide (112 mg, 2 mmol) were added in an ice bath. The reaction was carried out at room temperature overnight. The reactants were added with saturated salt water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain Compound 21 (66 mg) in 32% yield. LCMS (ESI +): 411.03 (M + H)⁺.

### Example 24: Synthesis of Compound 22

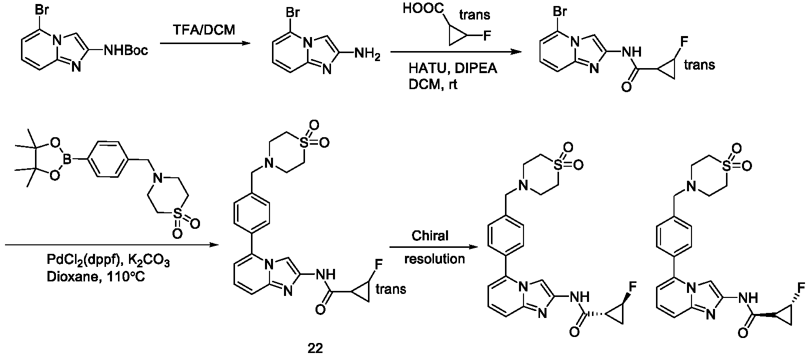

Step 1: *tert*-butyl(5-bromoimidazo[1,2-a]pyridin-2-yl)carbamate (311 mg, 1 mmol) was dissolved in dichloromethane (10 mL), and then trifluoroacetic acid (5 mL) was added in an ice bath. The reaction was conducted at room temperature for five hours before concentration. The resulting residue was used directly in the next step.

Step 2: The residue obtained step 1 was dissolved in dichloromethane (10 mL), followed by adding *trans*-2-fluorocyclopropanecarboxylic acid (114 mg, 1.1 mmol), and then N, N-diisopropylethylamine (260 mg, 2 mmol) and 2-(7-Azabenzotriazol-1-yl)-N,N,N',N'-tetramethyluronium hexafluorophosphate (570 mg, 1.5 mmol) were added in an ice bath. The reaction was conducted at room temperature overnight. After the reaction was completed, the reactants were added with water (20 mL) and then extracted with dichloromethane (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain a compound *trans*-N-(5-bromoimidazo[1,2-a]pyridin-2-yl)-2-fluorocyclopropanecarboxamide.

Step 3: *Trans*-N-(5-bromoimidazo[1,2-a]pyridin-2-yl)-2-fluorocyclopropanecarboxamide (149 mg, 0.5 mmol), 4-[4-(4,4,5,5-Tetramethyl[1,3,2]dioxaborolan-2-yl)benzyl]thiomorpholine 1,1-dioxide (175 mg, 0.5 mmol) , [1,1'-bis(diphenylphosphino)ferrocene]palladium dichloride (36 mg, 0.05 mmol) and potassium carbonate (138 mg, 1 mmol) were dissolved in dioxane (10 mL) and water (10 mL). The reaction material was subject to 3 times of nitrogen displacement and then stirred at 110 °C for 8 hours. After restored to room temperature, the reactants were added with water (20 mL) and then extracted with ethyl acetate (10 mL × 3). The organic phases were then combined and washed with saturated salt water (20 mL), dried with anhydrous sodium sulfate, filtered and concentrated under reduced pressure. The resulting residue was purified by column chromatography (dichloromethane:methanol = 100:0-100:5) to obtain Compound 22 (124 mg) in 56% yield. LCMS (ESI+): 443.55 (M + H)⁺.

The resulting Compound 22 was then resolved under conditions as follows: firstly, Compound 22 was dissolved in dichloromethane (concentration: 10 mg/mL), and then diluted with ethanol to 2.5 mg/mL; Chiral column UniChiral CMD-5H (21.2 mm I.D × 250 mmL) was used, mobile phase: ethanol/diethylamine (100:1), flow rate: 12 mL/min, UV: 254 nm, column temperature: 25 °C; after being resolved, Compounds 22-1 and 22-2 were obtained, respectively.

Retention time: Compound 22-1: 10.69 min (> 99% ee), Compound 22-2: 13.54 min (> 99% ee).

### Example 25: Synthesis of Compound 23

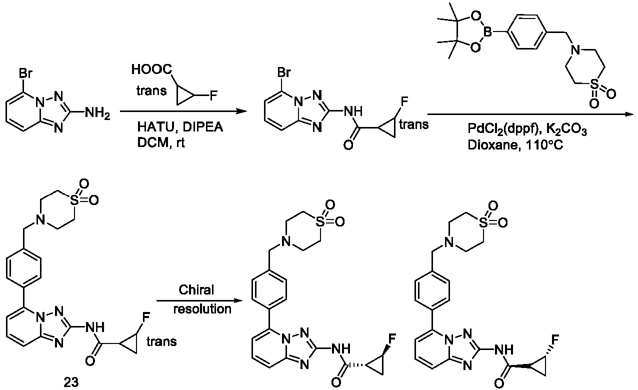

In accordance with the synthetic route of Example 24, *tert*-butyl(5-bromo-[1,2,4]triazolo[1,5-a]pyridin-2-yl)carbamate was used instead of *tert*-butyl (5-bromoimidazo[1,2-a]pyridin-2-yl)carbamate in Step 1 to obtain Compound 23 (100 mg) in 45% yield. LCMS (ESI +): 444.17 (M + H)⁺.

Then the resulting Compound 23 was resolved under conditions as follows: Compound 23 was dissolved in ethanol (4 mg/mL); Chiral column UniChiral CMD-5H (21.2 mm I.D × 250 mmL) was used, mobile phase: ethanol, flow rate: 12 mL/min, UV: 254 nm, column temperature: 25 °C; after being resolved, Compounds 23-1 and 23-2 were obtained, respectively.

Retention time: Compound 23-1: 11.58 min (> 99% ee), Compound 23-2: 16.47 min (> 99% ee).

### Example 26: Synthesis of Compounds 24-33

In accordance with the synthetic route of Examples 24 and 25, Compounds 24-33 can be synthesized with suitable raw materials.

Specifically, the numberings, structures, synthetic methods and characterization data of the compounds are as follows:

| Compound No. | Compound structure | Synthetic method | LCMS (ESI +)/ (M+H)⁺ |
|---|---|---|---|
| 24 | 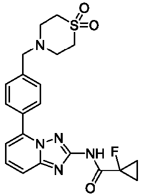 | See Example 25 | 444.21 |
| 25 | 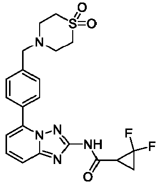 | See Example 25 | 462.18 |
| 26 | 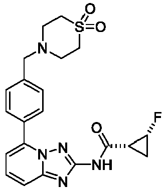 | See Example 25 | 444.18 |
| 27 | 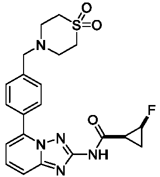 | See Example 25 | 444.18 |
| 28 | 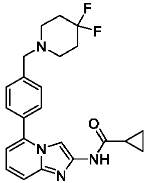 | See Example 24 | 411.53 |
| 29 | 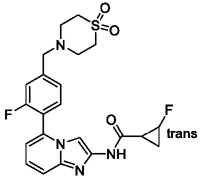 | See Example 24 | 461.12 |
| 30 | 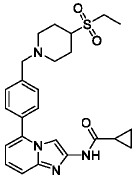 | See Example 24 | 467.20 |
| 31 | 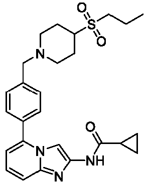 | See Example 24 | 481.21 |
| 32 | 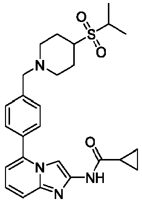 | See Example 24 | 481.21 |
| 33 | 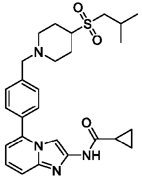 | See Example 24 | 495.22 |

### Example 27: JAK1/ JAK2/ JAK3 kinase inhibitory activity determination

### 1. Experimental consumables

| | | |
|---|---|---|
| JAK1: | Thermo Fisher PV4774 | 2059141D |
| JAK2: | Carna 08-045 | 14CBS-0374 H |
| JAK3: | Carna 08-046 | 19CBS-0798 B |
| ATP (10 mM): | CST 9804 | |
| DTT: | 100 mM | |
| MgCl₂: | 1 M | |
| MnCl₂: | 1 M | |
| HTRF Kinase-TK kit: | cis-bio 62TK0PEC | |
| HTRF 96 well low volume plate: | cis-bio 66PL96100 | |

### 2. Test conditions

JAK1: 0.32 ng/µL, ATP 4 µM, substrate 1 µM, time 4 h
JAK2: 0.008 ng/µL, ATP 4 µM, substrate 1 µM, time 2 h
JAK3: 0.1 ng/µL, ATP 3 µM, substrate 1 µM, time 3 h

### 3. JAK1 kinase inhibitory activity determination

### 3.1 Reagent preparation

1) Preparation of 1× kinase buffer: 5× kinase buffer was diluted to 1× kinase buffer with sterile water, then 5 mM MgCl₂, 1 mM MnCl₂, and 1 mM DTT were added;
2) Preparation of 5× JAK1: JAK1 mother liquor concentration: 160 ng/µL, which was subjected to 100-fold dilution to give the final concentration 5×, i.e., 1.6 ng/µL.
3) Preparation of 5× ATP: from 4 µM ATP to its 5×, i.e., 20 µM: the desired ATP concentration was obtained by 500-fold dilution of 10 mM ATP.
4) Preparation of 5× substrate: 5× 1 µM = 5 µM, substrate concentration: 500 µM, which was subjected to 100-fold dilution to give 5 µM substrate.
5) Preparation of 2.5× compound to be tested: the compound buffer concentration: 10 mM, and the treatment concentration started from 10 µM, which was prepared to 100× (i.e., 1 mM) buffer by 10× dilution of 10 mM, followed by a 1:3 gradient dilution to give a total of 10 concentrations; 2 µL of the diluted compound was taken and added to 78 µL of 1× kinase buffer to give 2.5 × compound to be tested; 2 µL of dimethyl sulfoxide (DMSO) was added to the positive control and blank control groups.
6) Preparation of 1 µM Streptavidin-XL665: Streptavidin-XL665 concentration: 16.67 µM, which was subjected to 16.67-fold dilution with the detection buffer during the use.
7) Preparation of 1× TK-Antibody: TK-Antibody stock solution: 100× solution, which was diluted to 1× with the detection buffer during the use.

### 3.2 Test method

1) 4 µL of the 2.5× compound to be tested was added to an HTRF 96 well low volume plate, and then 2 µL of 5× substrate was added from one side of the wells, 2 µL of 5× JAK1 was added from the other side of the wells; 2 µL of 1× kinase buffer was added in the blank control group;
2) the plate was sealed with a plate sealing film and placed in a centrifuge for centrifuging at 1000 rpm for 2 minutes;
3) 2 µL of 5× ATP was added to each well, and the plate was sealed with a plate sealing film, centrifuged at 1000 rpm for 1 min, and then put into an incubator at 30 °C for incubating 4 h;
4) at the end of incubation, 1 µM Streptavidin-XL665 and 1× TK-Antibody described above were mixed at a ratio of 1:1, then added to each well with a volume of 10 µL; the plate was centrifuged at 1000 rpm for 1 minute;
5) the plate was then put back into the incubator for incubating another 1 h; at the end of incubation, HTRF665/620 signals were read on a multifunctional microplate reader.

### 4. JAK2 kinase inhibitory activity determination

### 4.1 Reagent preparation

1) Preparation of 1× kinase buffer: 5× kinase buffer was diluted to 1× kinase buffer with sterile water, then 5 mM MgCl₂, and 1 mM DTT were added.
2) Preparation of 5× JAK2: JAK2 mother liquor concentration: 166 ng/µL, which was diluted to 1.66 ng/µL, and then subjected to 41.5-fold dilution to give the final concentration 5×, i.e., 0.04 ng/µL.
3) Preparation of 5× ATP: from 4 µM ATP to its 5×, i.e., 20 µM: the desired ATP concentration was obtained by 500-fold dilution of 10 mM ATP.
4) Preparation of 5× substrate: 5× 1 µM = 5 µM, substrate concentration: 500 µM, which was subjected to 100-fold dilution to give 5 µM substrate.
5) Preparation of 2.5× compound to be tested: the compound buffer concentration: 10 mM, and the treatment concentration started from 10 µM, which was prepared to 100× (i.e., 1 mM) buffer by 10× dilution of 10 mM, followed by a 1:3 gradient dilution to give a total of 10 concentrations; 2 µL of the diluted compound was taken and added to 78 µL of 1× kinase buffer to give 2.5 × compound to be tested; 2 µL of dimethyl sulfoxide (DMSO) was added to the positive control and blank control groups.
6) Preparation of 1 µM Streptavidin-XL665: Streptavidin-XL665 concentration: 16.67 µM, which was subjected to 16.67-fold dilution using the detection buffer.
7) Preparation of 1× TK-Antibody: TK-Antibody stock solution: 100× solution, which was diluted to 1× with the detection buffer during the use.

### 4.2 Test method

1) 4 µL of the 2.5× compound to be tested was added to an HTRF 96 well low volume plate, and then 2 µL of 5 × substrate was added from one side of the wells, 2 µL of 5 × JAK2 was added from the other side of the wells; 2 µL of 1× kinase buffer was added in the blank control group;
2) the plate was sealed with a plate sealing film and placed in a centrifuge for centrifuging at 1000 rpm for 2 minutes;
3) 2 µL of 5× ATP was added to each well, and the plate was sealed with a plate sealing film, centrifuged at 1000 rpm for 1 min, and then put into an incubator at 30 °C for incubating 2 h;
4) at the end of incubation, 1 µM Streptavidin-XL665 and 1× TK-Antibody described above were mixed at a ratio of 1:1, then added to each well with a volume of 10 µL; the plate was centrifuged at 1000 rpm for 1 minute;
5) the plate was then put back into the incubator for incubating another 1 h; at the end of incubation, HTRF665/620 signals were read on a multifunctional microplate reader.

### 5. JAK3 kinase inhibitory activity determination

### 5.1 Reagent preparation

1) Preparation of 1× kinase buffer: 5× kinase buffer was diluted to 1× kinase buffer with sterile water, then 5 mM MgCl₂, and 1 mM DTT were added.
2) Preparation of 5× JAK3: JAK3 mother liquor concentration: 124 ng/µL, which was subjected to 248-fold dilution to give the final concentration 5×, i.e., 0.5 ng/µL.
3) Preparation of 5× ATP: from 3 µM ATP to its 5×, i.e., 15 µM: the desired ATP concentration was obtained by 666.67-fold dilution of 10 mM ATP.
4) Preparation of 5× substrate: 5× 1 µM = 5 µM, substrate concentration: 500 µM, which was subjected to 100-fold dilution to give 5 µM substrate.
5) Preparation of 2.5× compound to be tested: the compound buffer concentration: 10 mM, and the treatment concentration started from 10 µM, which was prepared to 100× (i.e., 1 mM) buffer by 10× dilution of 10 mM, followed by a 1:3 gradient dilution to give a total of 10 concentrations; 2 µL of the diluted compound was taken and added to 78 µL of 1× kinase buffer to give 2.5 × compound to be tested; 2 µL of dimethyl sulfoxide (DMSO) was added to the positive control and blank control groups.
6) Preparation of 1 µM Streptavidin-XL665: Streptavidin-XL665 concentration: 16.67 µM, which was subjected to 16.67-fold dilution with the detection buffer during the use.
7) Preparation of 1× TK-Antibody: TK-Antibody stock solution: 100× solution, which can be diluted to 1× with the detection buffer during the use.

### 5.2 Test method

1) 4 µL of the 2.5× compound to be tested was added to an HTRF 96 well low volume plate, and then 2 µL of 5 × substrate was added from one side of the wells, 2 µL of 5 × JAK3 was added from the other side of the wells; 2 µL of 1× kinase buffer was added in the blank control group;
2) the plate was sealed with a plate sealing film and placed in a centrifuge for centrifuging at 1000 rpm for 2 minutes;
3) 2 µL of 5× ATP was added to each well, and the plate was sealed with a plate sealing film, centrifuged at 1000 rpm for 1 min, and then put into an incubator at 30 °C for incubating 3 h;
4) at the end of incubation, 1 µM Streptavidin-XL665 and 1× TK-Antibody described above were mixed at a ratio of 1:1, then added to each well with a volume of 10 µL; the plate was centrifuged at 1000 rpm for 1 minute;
5) the plate was then put back into the incubator for incubating another 1 h. At the end of incubation, HTRF665/620 signals were read on a multifunctional microplate reader.

### 6. Calculation of inhibition rate and fitting of IC₅₀

Inhibition rate = (Positive control group - compound to be tested)/(Positive control group - blank control group) × 100%

The half-maximal inhibitory concentration (IC₅₀) was fitted using GraphPad Prism 6 based on the kinase inhibition rates of the compound to be tested at different concentrations.

The representative compounds of the present application as well as the known control drug Filgotinib were tested for their inhibitory activity against JAK1, JAK2 and JAK3 kinases by the above determinations and the measured IC₅₀ values are shown in Table 1 below.

**Table 1 IC₅₀ of JAK1, JAK2 and JAK3 kinase activity inhibition by the representative compounds of the present application and Filgotinib**

| Compound No. | JAK1 IC₅₀ (nM) | JAK2 IC₅₀ (nM) | JAK3 IC₅₀ (nM) |
|---|---|---|---|
| 1 | 57.6 | 4.13 | 55.8 |
| 2 | 77 | 13.5 | 212 |
| 3 | 37.8 | 6.87 | 66.5 |
| 4 | 19.5 | 4.68 | 36.3 |
| 5 | 14 | 1.81 | 33.8 |
| 6 | 48.9 | 9.49 | 52.4 |
| 7 | 7.62 | 1.72 | 14.2 |
| 8 | 26.6 | 2.41 | 38.1 |
| 9 | 28.3 | 13.4 | 51 |
| 10 | 6.64 | 1.24 | 13 |
| 11 | 36.6 | 6.28 | 115 |
| 12 | 10 | 0.7 | 37.7 |
| 13 | 8.4 | 0.8 | 46.8 |
| 14 | 115.3 | 6.7 | 560.4 |
| 15 | 137 | 232 | 3175.1 |
| 16 | 74.4 | 127.1 | 3118.5 |
| 17 | 125 | 72.7 | 1455.3 |
| 19 | 182 | 90.3 | 909 |
| 20 | 262 | 176.4 | 5761 |
| 21 | 247 | 592 | 5966.8 |
| 22-1 | 117.4 | 334.3 | 8954.7 |
| 22-2 | 279.4 | 101.9 | 5120 |
| 23-1 | 145 | 1592 | >10000 |
| 23-2 | 199 | 873 | >10000 |
| 24 | 3640 | 1580 | >10000 |
| 25 | 590 | 1980 | 7780 |
| 26 | 1250 | 1090 | 3450 |
| 27 | 1080 | 415 | 1920 |
| 28 | 26.2 | 24.2 | 94.9 |
| 29 | 88.7 | 169.6 | 6111.4 |
| 30 | 387.0 | 162.0 | 5593.9 |
| 31 | 611.1 | 229.1 | 6901.3 |
| 32 | 955.5 | 448.1 | 9638.3 |
| 33 | 675.1 | 463.4 | 8279.8 |
| Filgotinib | 130 | 118 | 942 |

As seen from Table 1, the compounds of the present application have good JAK1, JAK2 or JAK3 kinase inhibitory activity and have significant selectivity for JAK1, JAK2 or JAK3 kinases compared to the known control drug Filgotinib.

### Example 28: Pharmacokinetic determination

### 1. Experimental animal

Three healthy male C57 mice, aged 6-8 weeks, were purchased from Shanghai Sippr-BK Laboratory Animal Co. Ltd. (Shanghai, China).

### 2. Experimental method

Mice were fasted overnight prior to oral administration, and food supply was resumed 4 h after administration with free access to water. Whole blood samples were collected from mice administered by gavage at 10 mg compound/kg body weight by way of facial vein semi-serial blood sampling; approximately 30 µL of blood was collected from the tested animals at 0.125 h, 0.25 h, 0.5 h, 1 h, 2 h, 4 h, 8 h, and 24 h, respectively, after administration and placed in test tubes containing the anticoagulant sodium heparin, which were placed on ice until centrifugation, and within 15 min, centrifuged in a centrifuge at 6800 g for 6 min at 6-8 °C, and within 1 h of blood collection/centrifugation, plasma was transferred to appropriately labeled tubes and stored frozen at about -80 °C.

### 3. Chromatography and mass spectrometry conditions

Chromatographic column: Luna^{®}Omega ACQUITY UPLC BEH C18 (2.1×50 mm, 1.7 µm); mobile phase A: H₂O-0.1 %FA; mobile phase B: ACN-0.1 %FA; flow rate: 0.80 mL/min; gradient elution program: starting from 10 % B; 0.6 min, 10 % B; 1.0 min, 90 % B; 1.11 min, 90 % B; 1.40 min, 10 % B; column temperature: 40 °C; injection volume: 2 µL;
Mass spectrometry method: LC-MS/MS-19 (TQ5500) (SCIEX, USA), ion source: ESI source, detection mode: positive ion detection, scanning mode: multi-reaction monitoring (MRM) mode, m/z: 271.10/172.00 Da (Tolbutamide, internal standard).

### 4. Preparation of plasma sample

To 10 µL of the plasma sample, was added 200 µL of internal standard working solution (Tolbutamide, 100 ng / mL), with vortex for 1 min, followed by centrifugation for 10 min at 18,000 g. 200 µL of a supernatant was transferred to a 96-well low volume plate, and 1 µL of the supernatant was taken and injected into LC-MS/MS for analysis.

### 5. Analysis of results

Pharmacokinetic (PK) parameters were calculated using Phoenix WinNonlin 7.0 software, and oral pharmacokinetic parameters of mice including AUC, Cₘₐₓ, Tₘₐₓ, T1/2, etc., were estimated by the non-compartment model. The results of oral pharmacokinetic parameters of mice for the representative compound 15 of the present application and the known control drug Filgotinib are shown in Table 2.

**Table 2 Oral pharmacokinetic parameters for compound 15 of the present application and Filgotinib**

| Compound No. | Dose mg/kg | T1/2 (h) | Tₘₐₓ (h) | Cₘₐₓ (ng/mL) | AUC₍₀₋ₜ₎ (h*ng/mL) | AUC_{(0-∞)} (h*ng/mL) | MRT₍₀₋t) (h) | MRT(0-∞) (h) |
|---|---|---|---|---|---|---|---|---|
| 15 | 10 | 1.31 | 0.29 | 6219.10 | 9747.53 | 9875.54 | 1.60 | 1.70 |
| Filgotinib | 5 | 1.79 | 0.250 | 1714.43 | 2121.28 | 2190.26 | 1.54 | 1.82 |

As can be seen from Table 2, the unit dose of compound 15 of the present application has a higher systemic exposure (AUC) compared to the known control drug Filgotinib, thus providing a significant pharmacokinetic advantage.

Preferred embodiments of the present application are described in detail above, however, the present application is not limited to the specific details of the above embodiments, within the scope of the technical conception of the present application, a variety of simple variants of the technical solution of the present application may be performed, these simple variants fall within the scope of protection of the present application.

It is also to be noted that the various specific technical features described in the above specific embodiments may be combined in any suitable manner without contradiction, and in order to avoid unnecessary repetition, the various possible combinations will not be separately described in the present application.

Further, any combination may be made between the various embodiments of the present application, as long as it does not contravene the ideas of the present application, it shall likewise be regarded as the disclosed content of the present application.

### Industrial applicability

The present application provides cyclopropanecarboxamide-containing compounds, preparation methods therefor and uses thereof. The cyclopropanecarboxamide-containing compounds provided in the present application have good inhibitory activity and obvious selectivity for JAK1, JAK2 or JAK3 kinases, and have good pharmacokinetic characteristics, which have great medical value and market potential for diseases associated with abnormal JAK signaling pathway (e.g., autoimmune diseases and myeloproliferative neoplasm diseases).

## Claims

1. A compound of formula (I), a stereoisomer, or a pharmaceutically acceptable salt thereof: 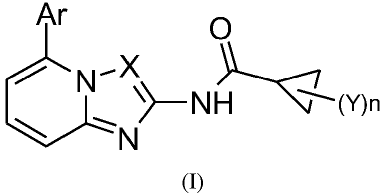 wherein,
X is selected from -CH or N;
Y is selected from hydrogen or halogen, and n is 1 or 2;
Ar is selected from 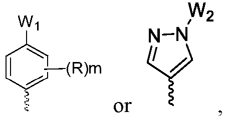 wherein:
m is an integer from 1-4;
Wi is selected from 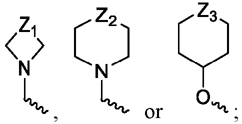
Wz is selected from 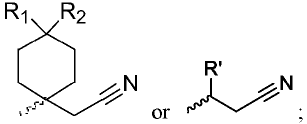
R is selected from hydrogen, C₁-C₆ alkyl, C₁-C₆ alkoxy, or halogen;
Z₁, Z₂ and Z₃ are each independently selected from -C(R₃R₄)-, -S(O)₂-, -S(O)-, -CH(S(O)₂R')-, or - CH(S(O)₂NR'R")-;
R₁ and R₂ are each independently selected from hydrogen, halogen, C₁-C₆ alkyl, C₁-C₆ haloalkyl, or C₃-C₇ cycloalkyl;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

2. The compound of formula (I), the stereoisomer, or the pharmaceutically acceptable salt thereof according to claim 1, having a structure of formula (II): 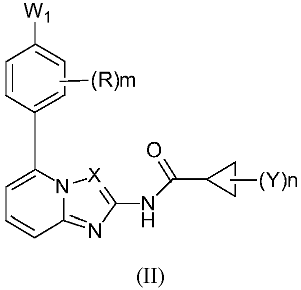 wherein,
X is selected from -CH or N;
Y is selected from hydrogen or halogen, and n is 1 or 2;
m is an integer from 1-4;
Wi is selected from 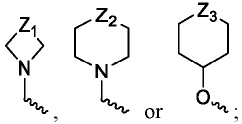
R is selected from hydrogen, C₁-C₆ alkyl, C₁-C₆ alkoxy, or halogen;
Z₁, Z₂ and Z₃ are each independently selected from -C(R₃R₄)-, -S(O)₂-, -S(O)-, -CH(S(O)₂R')-, or - CH(S(O)₂NR'R")-, and when Y is hydrogen, Z₂ is not -S(O)z-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

3. The compound of formula (I), the stereoisomer, or the pharmaceutically acceptable salt thereof according to claim 1, having a structure of formula (III): 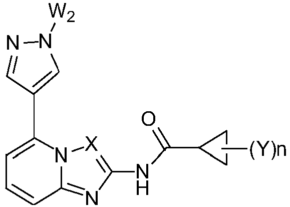 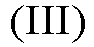 wherein,
X is selected from -CH or N;
Y is selected from hydrogen or halogen, and n is 1 or 2;
Wz is selected from 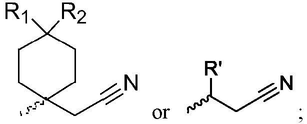
R₁ and R₂ are each independently selected from hydrogen, halogen, C₁-C₆ alkyl, C₁-C₆ haloalkyl, or C₃-C₇ cycloalkyl;
R' is selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂.

4. The compound of formula (I), the stereoisomer, or the pharmaceutically acceptable salt thereof according to claim 2, having a structure of formula (IV): 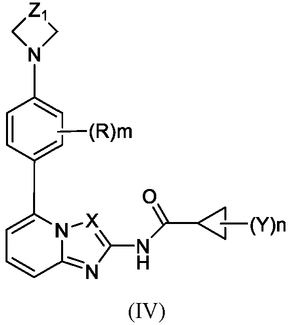 wherein,
X is selected from -CH or N;
Y is selected from hydrogen or halogen, and n is 1 or 2;
m is an integer from 1-4;
R is selected from hydrogen, C₁-C₆ alkyl, C₁-C₆ alkoxy, or halogen;
Z₁ is selected from -C(R₃R₄)-, -S(O)₂-, -S(O)-, -CH(S(O)₂R')- , or -CH(S(O)₂NR'R")-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

5. The compound of formula (I), the stereoisomer, or the pharmaceutically acceptable salt thereof according to claim 2, having a structure of formula (V): 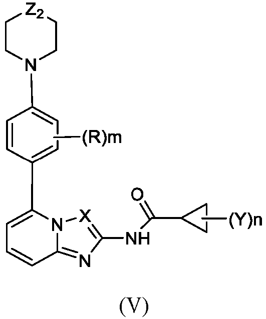 wherein,
X is selected from -CH or N;
Y is selected from hydrogen or halogen, and n is 1 or 2;
m is an integer from 1-4;
R is selected from hydrogen, C₁-C₆ alkyl, C₁-C₆ alkoxy, or halogen;
Z₂ is selected from -C(R₃R₄)-, -S(O)₂-, -S(O)-, -CH(S(O)₂R')- , or -CH(S(O)₂NR'R")-, and when Y is hydrogen, Z₂ is not -S(O)₂-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

6. The compound of formula (I), the stereoisomer, or the pharmaceutically acceptable salt thereof according to claim 2, having a structure of formula (VI): 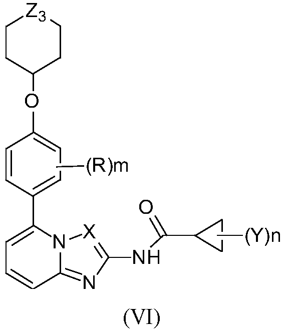 wherein,
X is selected from -CH or N;
Y is selected from hydrogen or halogen, and n is 1 or 2;
m is an integer from 1-4;
R is selected from hydrogen, C₁-C₆ alkyl, C₁-C₆ alkoxy, or halogen;
Z₃ is selected from -C(R₃R₄)-, -S(O)₂-, -S(O)-, -CH(S(O)₂R')-, or -CH(S(O)₂NR'R")-;
R' and R" are each independently selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂;
R₃ and R₄ are each independently selected from hydrogen, halogen, C₁-C₆ alkyl, C₁-C₆ haloalkyl, or C₃-C₇ cycloalkyl.

7. The compound of formula (I), the stereoisomer, or the pharmaceutically acceptable salt thereof according to claim 3, having a structure of formula (VII): 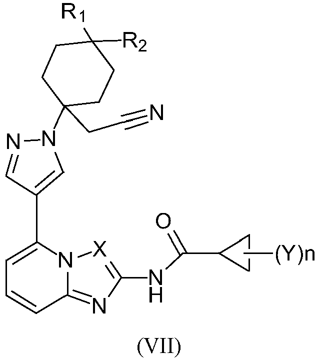 wherein,
X is selected from -CH or N;
Y is selected from hydrogen or halogen, and n is 1 or 2;
R₁ and R₂ are each independently selected from hydrogen, halogen, C₁-C₆ alkyl, C₁-C₆ haloalkyl, or C₃-C₇ cycloalkyl.

8. The compound of formula (I), the stereoisomer, or the pharmaceutically acceptable salt thereof according to claim 3, having a structure of formula (VIII): 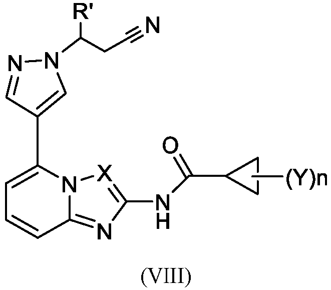 wherein,
X is selected from -CH or N;
Y is selected from hydrogen or halogen, and n is 1 or 2;
R' is selected from substituted or unsubstituted C₁-C₆ alkyl or C₃-C₇ cycloalkyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl, and N(C₁-C₆ alkyl)₂.

9. The compound of formula (I), the stereoisomer, or the pharmaceutically acceptable salt thereof according to any one of claims 1-8, wherein Y is selected from H or F.

10. The compound of formula (I), the stereoisomer, or the pharmaceutically acceptable salt thereof according to any one of claims 1-8, wherein 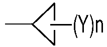 in the formulas is selected from

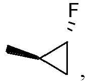

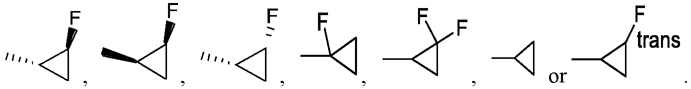

11. The compound of formula (I), the stereoisomer, or the pharmaceutically acceptable salt thereof according to claim 4, wherein, in formula (IV), Z₁ is selected from -CH(SO₂R'), R' is selected from unsubstituted C₁-C₆ alkyl or C₃-C₇ cycloalkyl; R is selected from hydrogen, methyl, methoxy , or halogen.

12. The compound of formula (I), the stereoisomer, or the pharmaceutically acceptable salt thereof according to claim 5, wherein, in formula (V), when Y is hydrogen, Z₂ is selected from -CR₃R₄- or -CH(S(O)₂R')-, and further, wherein, R₃ and R₄ are each independently selected from hydrogen, halogen, C₁-C₆ alkyl, C₁-C₆ haloalkyl, or C₃-C₇ cycloalkyl, R' is selected from C₁-C₆ alkyl or C₃-C₇ cycloalkyl, R is selected from hydrogen, methyl, methoxy, or halogen; when Y is halogen, Z₂ is selected from -SO₂-, and R is selected from hydrogen, methyl, methoxy, or halogen.

13. The compound of formula (I), the stereoisomer, or the pharmaceutically acceptable salt thereof according to claim 6, wherein, in formula (VI), Z₃ is selected from -SO₂- or -S(O)-; R is selected from hydrogen, methyl, methoxy, or halogen.

14. The compound of formula (I), the stereoisomer, or the pharmaceutically acceptable salt thereof according to claim 7, wherein, in formula (VII), R₁ and R₂ are each independently selected from hydrogen, fluorine, methyl, ethyl, *n*-propyl, isopropyl, *n*-butyl, isobutyl, *tert*-butyl, *sec*-butyl, *n-*pentyl, trifluoromethyl, cyclopropyl, or cyclopentyl.

15. The compound of formula (I), the stereoisomer, or the pharmaceutically acceptable salt thereof according to claim 8, wherein, in formula (VIII), R' is selected from substituted or unsubstituted methyl, ethyl, *n*-propyl, isopropyl, *n*-butyl, isobutyl, *tert*-butyl, *sec*-butyl, *n*-pentyl, isopentyl, cyclopropyl, or cyclopentyl, the substituent is selected from the group consisting of: halogen, C₁-C₆ alkoxy, C₁-C₆ alkylthio, C₃-C₇ cycloalkyl, hydroxyl, amino, NHC₁-C₆ alkyl and N(C₁-C₆ alkyl)₂.

16. Compounds of the following formulas, stereoisomers or pharmaceutically acceptable salts thereof:

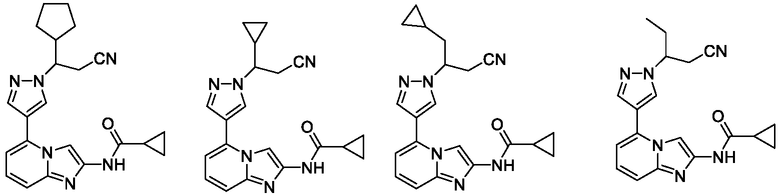

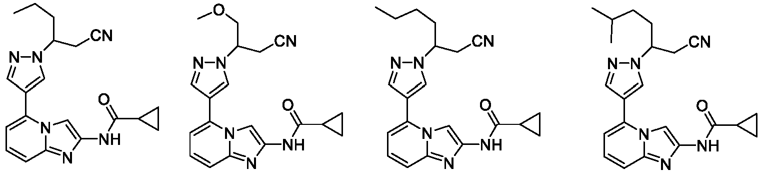

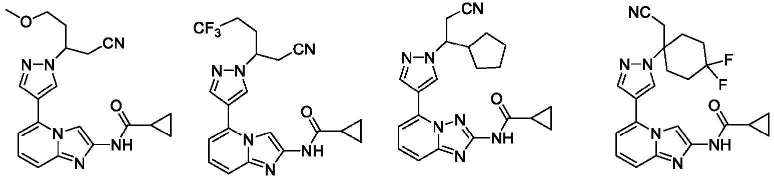

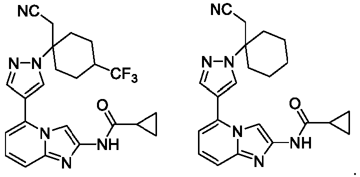

17. Compounds of the following formulas, stereoisomers or pharmaceutically acceptable salts thereof:

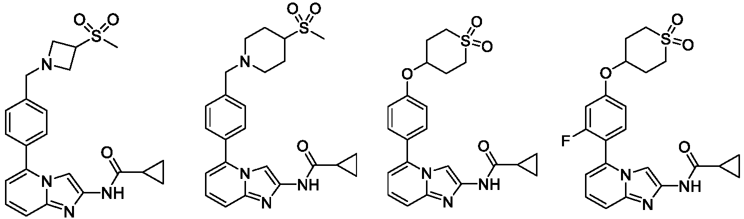

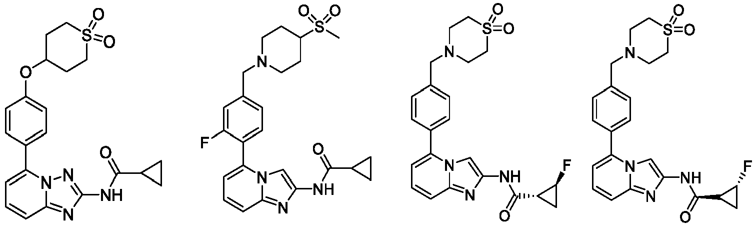

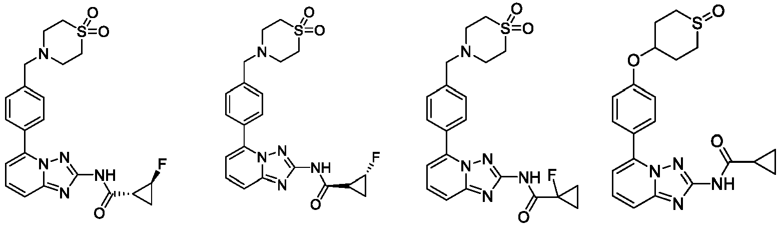

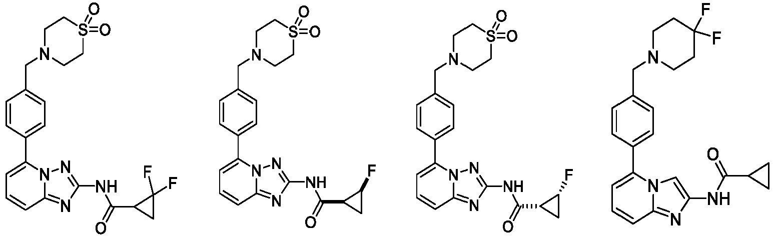

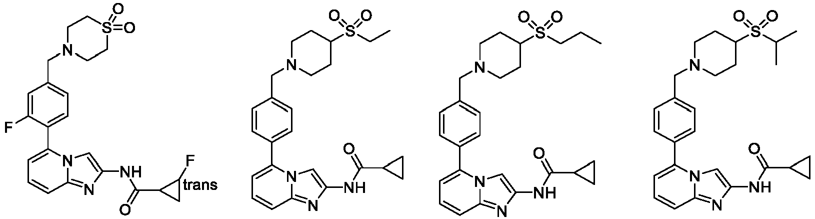

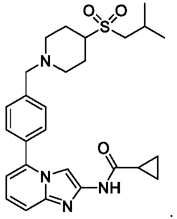

18. A pharmaceutical composition, comprising a therapeutically effective amount of the compound, the stereoisomer, or the pharmaceutically acceptable salt thereof according to any one of claims 1-17, and a pharmaceutically acceptable carrier.

19. Use of the compound, the stereoisomer, or the pharmaceutically acceptable salt thereof according to any one of claims 1-17, or the pharmaceutical composition according to claim 18 in the preparation of a medicament for treating a disease associated with abnormal JAK signaling pathway.

20. The use according to claim 19, wherein the disease is an autoimmune disease;
preferably, the autoimmune disease is selected from rheumatoid arthritis, ulcerative colitis, atopic dermatitis, or systemic lupus erythematosus.

21. The use according to claim 19, wherein the disease is a myeloproliferative neoplasm disease; preferably, the myeloproliferative neoplasm disease is selected from essential thrombocytosis, polycythemia vera, or primary myelofibrosis diseases.
